# DEMANDE DE BREVET EUROPEEN

(11) **EP 3 603 489 A1**
(43) Date de publication de la demande: **05.02.2020**
(21) Numéro de dépôt: 18306066.4
(22) Date de dépôt: 03.08.2018
(51) Int. Cl.: A61B 5/00, A61B 5/145

(54) **SYSTÈME DE SURVEILLANCE CORPORELLE AVEC LIAISON SÉPARABLE**

(71) Demandeur: PKvitality, 75001 Paris (FR)
(72) Inventeur: PIERART, Luc, 94800 VILLEJUIF (FR)
(74) Mandataire: Regimbeau

(57) **Abrégé**

L'invention propose un système de surveillance corporelle (1), destiné à être attaché à un membre d'un être vivant, comprenant :
- un premier module (100), comprenant des moyens d'attache et de serrage (110) configurés pour tenir et serrer le système (1) sur un membre,
- un deuxième module (200), comprenant des microaiguilles (210) configurées pour être insérées dans la peau pour prélever et/ou analyser un fluide corporel du porteur du système de surveillance corporelle (1) lorsque ce dernier est positionné sur le membre,
les deux modules (100, 200) pouvant être en position couplée entre eux par une complémentarité de forme,
le système étant caractérisé en ce que le première module (100) et le deuxième module (200) sont reliés entre eux par une liaison séparable (300), la liaison séparable étant configurée pour se libérer lorsqu'un seuil d'arrachement est dépassé, la liaison étant en outre réutilisable.

## Description

### DOMAINE TECHNIQUE GENERAL

La présente invention concerne un système de surveillance corporelle via analyse de liquide corporel, typiquement interstitiel, à l'aide de microaiguilles.

Plus précisément, la présente invention concerne la gestion du maintien des microaiguilles dans la peau.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenus sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Ce dispositif portatif, typiquement au poignet, permet une mesure en continu et il suffit de changer la capsule pour changer de microaiguilles.

Toutefois, lorsqu'un tel dispositif est porté au poignet ou à un autre membre, il y a un risque d'arrachement des microaiguilles lorsque le dispositif est accroché par une manche, une porte, une chaise, etc. Cet arrachement n'est pas acceptable en matière de douleur et d'intégrité de la peau.

En outre, il est important que le dispositif soit facilement utilisable.

### PRESENTATION DE L'INVENTION

Pour répondre à certaines de ces problématiques, l'invention propose, dans un premier aspect, un système de surveillance corporelle, destiné à être attaché à un membre d'un être vivant, comprenant :
- un premier module, comprenant des moyens d'attache et de serrage configurés pour tenir et serrer le système sur un membre,
- un deuxième module, comprenant des microaiguilles configurées pour être insérées dans la peau pour prélever et/ou analyser un fluide corporel du porteur du système de surveillance corporelle lorsque ce dernier est positionné sur le membre,
les deux modules pouvant être en position couplée entre eux par une complémentarité de forme,
le système étant caractérisé en ce que le première module et le deuxième module sont reliés entre eux par une liaison séparable, la liaison séparable étant configurée pour se libérer lorsqu'un seuil d'arrachement est dépassé, la liaison étant en outre réutilisable.

La liaison séparable se sépare par arrachement, c'est-à-dire un mouvement essentiellement de translation. Le seuil d'arrachement est faible. Par exemple, une valeur comprise entre 30 et 400g de seuil d'arrachement convient (en masse) ou une valeur comprise entre 0,3 et 4N convient (en poids), c'est-à-dire qu'une masse ou poids supérieur au seuil prédéfini appliqué en arrachement permet de séparer les deux modules. Préférablement, une valeur comprise entre 70 et 250g ou 0.7 et 2.5N est choisie.

En particulier, le système de surveillance corporelle est configuré pour passer :
- d'une position couplée, dans laquelle la première module et la deuxième module sont solidaires entre elles grâce à la liaison séparable, et peuvent échanger des signaux électriques et/ou du liquide,
- à une position libre, dans laquelle la première module et la deuxième module ne sont pas en position couplée.

Avantageusement, le seuil d'arrachement de la liaison séparable est inférieur au seuil d'arrachement des microaiguilles de la peau, de sorte qu'une fois porté, un effort d'arrachement sépare la liaison séparable pour éviter l'arrachement des microaiguilles de la peau.

Dans un mode de réalisation, le premier module comprend en outre un boitier, auquel sont attachés les moyens d'attache et de serrage.

Dans une variante les moyens d'attache et de serrage comprennent un réceptacle pour le boitier, le boitier étant attachable de façon amovible dans le réceptacle.

Dans un mode de réalisation, le boitier est attachable au deuxième module par la liaison séparable.

Dans un mode de réalisation, le deuxième module comprend une capsule, qui comprend elle-même les microaiguilles et comprend un patch attachable à la capsule. Le patch a alors un pouvoir adhésif à la peau (soit par son matériau propre, soit par l'ajout d'une couche adhésive).

Dans un mode de réalisation le patch est solidarisable du premier module par la liaison séparable.

Dans un mode de réalisation le patch est solidaire de la capsule de façon amovible, afin de pouvoir changer de patch en gardant la même capsule.

Dans un mode de réalisation le patch a une forme annulaire entourant la capsule, la capsule comprenant une protubérance configurée pour coopérer avec une encoche du patch.

Dans un mode de réalisation le seuil d'arrachement des microaiguilles tient compte de la fonction adhésive du patch.

Dans un mode de réalisation, les moyens d'attache et de serrage comprennent une lanière, par exemple en matériau élastique et/ou par exemple sous la forme d'un bracelet ajustable.

Dans un mode de réalisation la lanière est prise en sandwich entre la capsule et le boitier.

Dans un mode de réalisation la lanière est indépendante du boitier et permet d'assurer le serrage au membre indépendamment du boitier

Dans un mode de réalisation la liaison séparable comprend un aimant. L'aimant est situé dans le premier ou le deuxième module, voire dans les deux (pour une liaison plus fort).

Dans un mode de réalisation la liaison séparable comprend un clipsage.

Dans un mode de réalisation, le système comprend en outre un guide entre le premier module et le deuxième module, le guide étant configuré pour permettre un retour naturel en position couplée.

Le guide peut comprendre une forme tronconique compris dans le premier module et une ouverture complémentaire en entonnoir dans le deuxième module, le guide permettant de définir une position intermédiaire. La forme tronconique est alors partiellement ou totalement engagée dans l'ouverture complémentaire en entonnoir.

Dans un mode de réalisation, le calage entre le patch et le bracelet fait office de guide dans un plan, en empêchant la rotation (la partie tronconique permettant le guidage dans un plan orthogonal mais sans nécessairement bloquer la rotation selon cet axe).

Dans un mode de réalisation, le boitier comprend une forme tronconique (préférablement de révolution) et la capsule comprend une ouverture traversante évasée, de forme complémentaire.

Dans un mode de réalisation le boitier comprend une batterie et/ou un processeur pour la gestion des données du capteur notamment.

Dans un mode de réalisation, le système comprend des connecteurs électriques complémentaires sur le premier module et la deuxième module (200). Alternativement, les échanges d'information entre les deux modules peut se faire sans fil, à l'aide d'une connexion sans fil entre les deux modules

Le premier aspect de l'invention propose aussi un kit de surveillance corporelle, comprenant un système de surveillance corporelle et un dock configuré pour recevoir, au moins partiellement, le premier module du dispositif de surveillance corporelle. La liaison entre le dock et le système de surveillance est préférablement une liaison séparable qui utilise des éléments de la liaison séparable entre le premier module et le deuxième module. Avantageusement, des aimants sont utilisés.

Selon un deuxième aspect de l'invention, il est proposé un système de surveillance corporelle, destiné à être attaché à un membre d'un être vivant, comprenant :
- un premier module, comprenant :
   un boitier comprenant une batterie et/ou un microprocesseur, et
   des moyens d'attache et de serrage configuré pour tenir et serrer le système sur le membre,
- un deuxième module, comprenant :
   une capsule comprenant des microaiguilles configurées pour être insérées dans la peau pour prélever et/ou analyser un fluide corporel du porteur du système de surveillance corporelle lorsque ce dernier est positionné sur le membre,
   un patch avec un pouvoir adhésif à la peau, dans lequel :
      - les deux modules sont détachables de façon amovible d'une position couplées entre eux par une complémentarité de forme,
      - le boitier est détachable de façon amovible des moyens d'attache et de serrage,
      - le patch est détachable de façon amovible de la capsule.

Préférablement, le premier et le deuxième module sont reliés entre eux par une liaison séparable configurée pour lâcher lorsqu'un seuil d'arrachement des microaiguilles est dépassé. La liaison séparable a été décrite précédemment.

Préférablement la liaison séparable se fait entre le patch et le boitier.

Les différents modes de réalisation présentés pour le second aspect s'appliquent au premier aspect.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre en vue éclatée un bracelet, un boitier, une capsule et un patch tel qu'utilisable dans le cadre de l'invention,
- La figure 2 illustre un schéma représentant un système comprenant un bracelet, un boitier, une capsule, et un patch solidaire de la capsule et attaché au boitier, selon un mode de réalisation de l'invention,
- La figure 3 illustre un schéma représentant un bracelet et un boitier, monté sur un dock (ou base) de rechargement.

### DESCRIPTION DETAILLEE

### Architecture générale

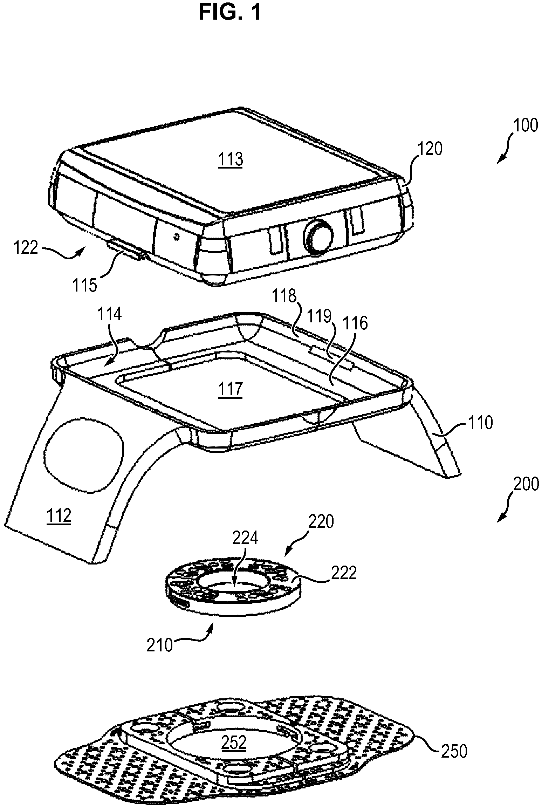

En référence aux **figures 1 à 3****,** la présente invention concerne un système électronique 1 de surveillance corporelle. Il s'agit d'une amélioration du dispositif du document WO2018104647. Par conséquent, l'invention se place dans le même concept général de système intégral autonome à faible douleur, faible risque hygiénique et réutilisable.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que le lactate, l'hydratation, etc.

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est destiné à être attaché à un membre d'un être vivant, typiquement un bras ou une jambe d'un être humain. La zone d'attache privilégiée est le poignet où le système 1 s'apparente à une montre.

Le système 1 est formé de deux modules 100, 200 reliées entre eux par une liaison séparable 300 qui est réutilisable. On définit ainsi une position couplée et une position libre. En position couplée, les deux modules 100, 200 ne sont pas séparés physiquement et peuvent échanger des données. Il est ainsi possible de manipuler le système comme un tout solidaire, pourvu qu'on n'exerce pas d'effort supérieur à un seuil d'arrachement, qui permet de désolidariser les deux modules 100, 200. En position couplée, il n'y a préférablement pas de liberté de mouvement possible entre les deux modules 100, 200.

Le premier module 100 comprend notamment des moyens d'attache 110 et de serrage 100 du système 1 à un membre et le deuxième module 200 comprend notamment des microaiguilles 210 configurées pour être insérées dans la peau (dans une partie superficielle de l'épiderme). Ces microaiguilles 210, lorsque le premier module 100 est en position sur le membre, permettent de prélever et/ou d'analyser un fluide corporel, comme mentionné précédemment. Les microaiguilles 210 sont disposés sur une face de contact d'une capsule 220 qui peut s'engager avec le premier module 100.

Les microaiguilles 210 consistent avantageusement en un réseau de microaiguilles 210 au contact de la peau lorsque le premier module 100 est placé sur le corps d'une personne. Les microaiguilles 210 peuvent donc être soit creuses, pour prélever du liquide, soit pleines, pour analyser directement le liquide. Dans le premier cas, typiquement, les microaiguilles 210 permettent l'extraction de liquide interstitiel du derme de façon indolore sans perlement de sang, et l'envoie vers un capteur présent dans le système 1 (plus précisément préférablement dans le deuxième module 200). Dans le deuxième cas, les microaiguilles 210 ne prélèvent pas de fluide et intègrent le capteur sur leur surface, sous la forme d'un matériau biochimique apte à réagir avec l'analyte que l'on souhaite mesurer dans le fluide.

De façon préférée, lesdits microaiguilles 210 comprennent entre quatre et cinquante microaiguilles, sensiblement pyramidales, avec des pointes d'une hauteur comprise entre 100µm et 1000µm, préférablement 0.3mm et 0.8mm. Chacune de ces caractéristiques avantageuses des microaiguilles peut être prise séparément ou en combinaison avec les autres.

Les deux modules 100 et 200 présentent chacun une face de couplage 122, 222, de forme complémentaire, qui permet de placer le deuxième module 200 dans un emplacement d'accueil du premier module 100. Ils sont donc en contact l'un avec l'autre.

Enfin, le premier module 100 et le deuxième module 200 sont configurés pour être couplés par la liaison séparable 300. Cette liaison 300 solidarise les deux modules 100, 200 tant qu'un seuil d'arrachement n'est pas dépassé. Une fois la liaison désactivée, elle peut être réactivée : il ne s'agit pas d'une liaison à utilisation unique, mais d'une liaison amovible, réutilisable un nombre indéfini de fois.

Ainsi, le deuxième module 200 est conçu pour être positionné sur la peau, avec les microaiguilles 210 qui restent insérées dans la peau, peu importe les mouvements du premier module 100 : soit ce sont des mouvements volontaires de séparation des deux modules 100, 200, soit ce sont des mouvements involontaires de la vie courante qui tendent à séparer les deux modules 100, 200 (blocage du premier module dans une porte, une manche, coup reçu involontairement, etc.). On évite ainsi l'arrachement des microaiguilles 210 dans toutes ces situations, puisque la liaison séparable 300 va lâcher avant que les microaiguilles 210 ne soient arrachées de la peau.

Comme illustré sur les **figures 1 à 3****,** le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran 113, éventuellement tactile pour afficher les résultats de la surveillance à l'utilisateur.

L'homme du métier connait des algorithmes de traitement de mesures de capteurs et des interfaces associées, et saura les implémenter dans le présent système 1.

Le boitier 120 est solidarisé aux moyens d'attache et de serrage 110 par clipsage par exemple (cf. infra.).

Le boitier 120 comprend en outre des connecteurs électriques, sur sa face de couplage 122 avec la face de couplage 222 de la capsule 220. Alternativement, la liaison se fait sans fil entre les deux modules (bluetooth, infrarouge, NFC, RFID, etc.).

Le deuxième module 200 peut comprendre une capsule 220 qui intègrent les microaiguilles 210. Cette capsule 220 a une forme de boite fermée, typiquement étanche, qui peut se coupler avec le boitier 120. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. Dans une variante mentionnée précédemment, le capteur est positionné à l'intérieur de la capsule 220 (ou alors dans le boitier 120) et analyse le fluide prélevé par les microaiguilles 210.

De la même façon, le deuxième module 200 peut comprendre un patch 250, solidaire de façon amovible à la capsule 220. Le patch 250 fonctionne comme un adhésif pour maintenir les microaiguilles 210 enfoncées dans la peau.

Dans le cas d'une liaison électrique avec le boitier 120, la capsule 220 comprend aussi des connecteurs électriques 226, sur la face de couplage 220 avec le boitier 120, qui peuvent coopérer avec les connecteurs électriques du boitier 120. S'il y a transmission de fluide depuis le deuxième module vers le premier alors des connecteurs fluidiques complémentaires sont prévus sur la capsule 220 et le boitier 120.

Le deuxième module 200 forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur, changer de capsule 220 permet de changer de capteurs si ceux-ci sont en fin de vie ou si l'on souhaite changer de grandeur physique mesurée, en une manipulation simple, rapide et sure, sans devoir jeter d'autres parties (en particulier le premier module). La capsule 220 a besoin d'être changée peu fréquemment (entre toutes les semaines et tous les mois, à titre d'exemple).

Dans la mesure où la capsule 220 minimise la quantité d'élément et/ou matériaux coûteux (équipement électronique avancé tel qu'une batterie ou des moyens de communication sans fil), elle est relativement peu chère.

### La liaison séparable 300

La liaison séparable 300 est conçue pour désolidariser les deux modules 100, 200, c'est-à-dire quitter la position couplée, dès qu'un seuil d'arrachement est dépassé.

En position couplée, le deuxième module 200 est logé dans l'emplacement complémentaire du premier module 100 et les deux modules 100, 200 peuvent échanger des données (essentiellement électriques grâce aux connecteurs électriques, mais éventuellement fluidiques). En position libre, le deuxième module 200 est hors de l'emplacement complémentaire. Lorsque la connexion électrique est physique (pas sans-fil donc), la connexion est rompue en position libre.

Le seuil d'arrachement correspond à celui à partir duquel un mouvement du système 1 entrainerait l'arrachement des microaiguilles 210. L'effort à fournir pour enlever les microaiguilles 210 dépend, outre ces dernières en elle-même, de la force adhésive du patch 250.

La liaison séparable 300 s'enclenche et se sépare par l'effet d'une translation. En effet, tout vissage pour enclencher la liaison séparable empêcherait la séparation de la liaison séparable 300 lors d'un arrachement involontaire.

La liaison séparable 300 forme une liaison faible. A cet égard, le seuil d'arrachement de la liaison séparable 300 est choisi entre 30 et 400g, préférablement entre 70 et 250g, ou bien entre 0,3 et 4N, préférablement entre 0,7 et 2,5N. L'arrachement est mesuré par l'application d'une masse ou d'une force à l'un des deux modules 100, 200 en direction orthogonale aux faces de couplages, l'autre module 200, 100 étant maintenu fixe (en particulier, la masse ou la force est appliqué à un des éléments intervenant dans la liaison séparable).

La liaison séparable se fait avantageusement entre le patch 250 et le premier module 100. Ainsi, les efforts d'arrachement vont directement par le patch 250, qui, en adhérant la peau, va les absorber sans les transmettre (ou alors faiblement) à la capsule 220 avec les microaiguilles 210.

Du côté du premier module 100, la liaison 300 peut se faire avec une lanière (ou un bracelet 112) des moyens 110, ou bien le boitier 120, sans préférence particulière.

Grâce à la liaison séparable 300, on peut définir au système 1 une position couplée, dans laquelle le premier module 100 et le deuxième module 200 sont en relation, avec notamment les connecteurs électriques qui sont en contact. S'il y a besoin de transmettre un fluide du deuxième module 200 vers le premier module 100, des connecteurs fluidiques sont prévus

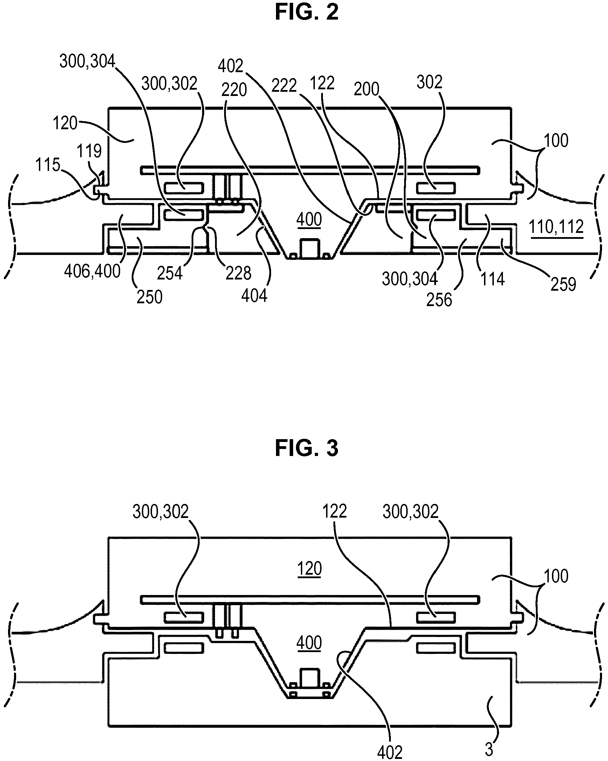

Dans un mode de réalisation, la liaison séparable 300 est réalisée par un aimant permanent 302. L'aimant 302 peut être positionné soit dans le premier module 100, soit dans le deuxième module 200. L'autre module comprend alors un matériau 304 attiré par l'aimant (de type ferromagnétique ou un autre aimant pour améliorer la force de maintien). Dans une variante illustrée sur la **figure 2****,** l'aimant 302 et le matériau 3004 ferromagnétique sont respectivement positionnés dans le boitier 120 et dans le patch 250 (ou inversement). L'aimant 302 et le matériau ferromagnétique 304, lorsque les deux modules 100, 200 sont couplés, se retrouvent en vis-à-vis.

Dans un mode de réalisation, la liaison séparable 300 est réalisée par un clipsage. On prévoit alors un picot (préférablement plusieurs) sur le premier ou le deuxième module 100, 200 et un orifice complémentaire sur l'autre module, qui autorise le clipsage du picot.

### Le patch 250 et la capsule 220

Le patch 250 comprend lui aussi une forme annulaire, avec un orifice traversant 252 au milieu à l'intérieur de laquelle se loge la capsule 220. Pour solidariser les deux, le patch 250 comprend avantageusement des encoches 254 au niveau de la paroi de l'orifice 252 pour recevoir des protubérances 228 de la capsule 220.

Autour de l'orifice 252, l'épaisseur du patch 250 est sensiblement égale (voir égale) à l'épaisseur de la capsule 220 (c'est ici qu'est avantageusement positionné une partie de la liaison amovible 300, comme l'aimant 302 ou le matériau 304). Le patch est d'ailleurs solide (rigide) dans cette zone. Dans une zone périphérique du patch 250, ce dernier est plus fin (souple) pour pouvoir épouser la forme du membre. En effet, dans le cas d'un poignet, la capsule 220 est positionnée sur une zone plutôt plate du membre, alors que les zones périphériques du patch 250 s'étendent jusqu'au courbure du poignet.

### Les guides 400

Avantageusement, le système comprend au moins un guide 400 configuré pour faciliter le retour naturel en position couplée des deux modules 100, 200. Le guide 400, dans certaines variantes, permet de définir une configuration intermédiaire, dans laquelle le deux modules 100, 200 ne sont pas couplés mais sont engagés via le guide : en d'autres termes, il y a toujours un contact physique qui se fait entre le deux modules 100, 200 par le biais du guide. En configuration intermédiaire, si l'effort d'arrachement cesse, le premier module 100 vient se remettre naturellement en position couplée grâce notamment aux moyens d'attache et de serrage 110 qui tendent à maintenir le premier module 100 au point en position intermédiaire. Lorsqu'on souhaite complètement enlever le boitier 120, il y a un désengagement complet du boitier 120 par rapport au deuxième module 200.

Dans un mode de réalisation, le guide 400 comprend un pan incliné 402, positionné sur le premier module 100, qui coopère avec un autre pan incliné 404 positionné sur le deuxième module 200. Pour des raisons de symétrie, les pans inclinés 402, 404 sont préférablement de révolution et forment une région tronconique.

Sur la **figure 2****,** le pan incliné 402 du premier module 100 est réalisé sous la forme d'une protubérance tronconique (comme un volcan) s'étendant depuis le boitier 120 (du côté qui reçoit la capsule 220) en se rétrécissant. Le pan incliné 404 du deuxième module 200 est réalisé par une ouverture évasée dans la capsule 220, de forme complémentaire. L'ouverture évasée peut être l'ouverture traversante 224 de la capsule 220, de sorte que le volcan vienne au contact de la peau : un capteur peut alors y être positionné (pouls, etc.). De plus, le pan incliné 402 peut être réalisé en matériau glissant (comme du verre par exemple) pour faciliter la remise en position couplée de façon naturelle. Ce guide 400 est structurellement indépendamment de la liaison séparable 300.

Dans un mode de réalisation non illustré, le guide est formé par un entonnoir autour de l'orifice qui reçoit le picot : il guide alors le picot vers l'orifice de clipsage.

Grâce au guide 400 et ses pans inclinés, lorsque le premier module 100 est arraché (et qu'il ne sort pas complètement du guide 400 naturellement), il se remet naturellement en position sur le deuxième module. Il fonctionne comme un guide mécanique tridimensionnel (en XZY).

Un autre guide est décrit ci-dessous.

### Moyens d'attache et de serrage 110

Ces moyens d'attache et de serrage 110 sont typiquement formés par une lanière qui peut être réglable ou élastique, par exemple sous forme d'un bracelet. On privilégie les moyens d'attache et de serrage 110 qui peuvent être réglés pour une meilleure gestion de la force d'appui exercée sur la capsule 220 (via le patch 250).

Dans un mode de réalisation préférentiel des moyens d'attache et de serrage 110, les moyens d'attache et de serrage 110 sont autonomes, c'est-à-dire qu'ils peuvent être montés sur le membre en l'absence des autres éléments (le boitier 120 principalement). Pour cela, les moyens d'attache et de serrage 110 comprennent une lanière 112 de longueur suffisante pour faire le tour du membre et un réceptacle 114 solidaire de la lanière à l'intérieur duquel se loge le boitier 120. Le réceptacle 114 divise la lanière 112 en deux branches, dont chacune est solidaire d'un côté opposé du réceptacle 114. Le réceptacle 114 est par exemple de forme généralement rectangulaire avec des angles arrondis.

Dans un mode de réalisation préférentiel d'agencement des éléments, le réceptacle 114 comprend une bordure interne 116 qui définit une ouverture 117, qui permet d'accueillir au moins une partie du deuxième module 200. La bordure interne 116 se loge entre le boitier 120 et le patch 250 et des parois 118. Côté boitier 120, la bordure interne 116 forme un fond partiel sur lequel se loge une périphérie externe du fond du boitier 120. La paroi 118 peut comprendre une encoche 119 (préférablement deux), à l'intérieur de laquelle se loge une languette 115 (préférablement deux) qui s'étend depuis le boitier 120. Côté patch 250, la bordure interne 116 forme, avec la lanière 112, une rainure périphérique dans lequel une partie de patch 250 se trouve, de sorte que la couronne interne 116 se loge entre le boitier 120 et le patch 250 et est ainsi prise en sandwich (**figure 2**).

La bordure interne 116 accueille donc une zone externe du patch (moins épaisse que la zone autour de l'orifice mais rigide, pour reprendre l'effort de la lanière 112) et fait ainsi office de guide 406 pour le replacement du deuxième module 200 sur le premier module 100 après que la liaison séparable 300 a été séparée. De la même façon, une portion interne du patch 250, plus épaisse que la portion externe, peut venir se loger à l'intérieur de la bordure 116. La paroi de la bordure 116 peut alors fait butée avec le joint et fait ainsi office de guide (en particulier selon un plan).

La lanière 112 et le réceptacle 114 peuvent être intégral.

Par ailleurs, le boitier 120 est positionné de façon amovible dans le réceptacle 114, de sorte que l'utilisateur puisse garder au poignet les différents éléments suivants :
- le deuxième module (200) seul,
- le deuxième module (200) et les moyens d'attache et de serrage (110), sans le boitier 120 (par exemple pour la piscine ou autre),
- le première module (100) seul (fonctionnement en montre connectée),
- les moyens d'attache et de serrage (110) seul (intérêt moindre).

### Le dock

Un dock de rechargement 3 peut être prévu, comme illustré en **figure 3****.** Le dock 3 forme une base pouvant recevoir le premier module 100, en se substituant au deuxième module 200. Le dock comprend l'autre partie de la liaison séparable 300, à l'instar du deuxième module 200.

Par amovible, on entend que l'on peut enlever ou remettre à volonté.

## Revendications

1. Système de surveillance corporelle (1), destiné à être attaché à un membre d'un être vivant, comprenant :
- un premier module (100), comprenant des moyens d'attache et de serrage (110) configurés pour tenir et serrer le système (1) sur un membre,
- un deuxième module (200), comprenant des microaiguilles (210) configurées pour être insérées dans la peau pour prélever et/ou analyser un fluide corporel du porteur du système de surveillance corporelle (1) lorsque ce dernier est positionné sur le membre,
les deux modules (100, 200) pouvant être en position couplée entre eux par une complémentarité de forme,
le système étant **caractérisé en ce que** le première module (100) et le deuxième module (200) sont reliés entre eux par une liaison séparable (300), la liaison séparable étant configurée pour se libérer lorsqu'un seuil d'arrachement est dépassé, la liaison étant en outre réutilisable.

2. Système de surveillance corporelle (1) selon la revendication 1, dans lequel le seuil d'arrachement de la liaison séparable (300) est inférieur au seuil d'arrachement des microaiguilles (210) de la peau, de sorte qu'une fois porté, un effort d'arrachement sépare la liaison séparable (300) pour éviter l'arrachement des microaiguilles (210) de la peau.

3. Système de surveillance corporelle (1) selon l'une quelconque des revendications 1 à 2, dans lequel le premier module (100) comprend en outre un boitier (120), auquel sont attachés les moyens d'attache et de serrage (110).

4. Système de surveillance selon la revendication 3, dans lequel le boitier (120) (120) est attachable au deuxième module (100) par la liaison séparable (300).

5. Système de surveillance corporelle (1) selon l'une quelconque des revendications 1 à 4, dans lequel le deuxième module (200) comprend une capsule (220), qui comprend elle-même les microaiguilles (210), et comprend un patch (250) attachable à la capsule (220), le patch (250) ayant un pouvoir adhésif à la peau.

6. Système selon la revendication 5, dans lequel le patch (250) est solidarisable du premier module (100) par la liaison séparable (300).

7. Système selon la revendication 5 ou 6, dans lequel le patch (250) est solidaire de la capsule (220) de façon amovible, afin de pouvoir changer de patch (250) en gardant la même capsule (220).

8. Système de surveillance corporelle (1) selon l'une quelconque des revendications précédentes, dans lequel les moyens d'attache et de serrage (110) comprennent une lanière (112), par exemple en matériau élastique et/ou par exemple sous la forme d'un bracelet ajustable.

9. Système de surveillance corporelle (1) selon l'une quelconque des revendications précédentes, dans lequel la liaison séparable (300) comprend un aimant (302).

10. Système de surveillance corporelle (1) selon l'une quelconque des revendications précédentes, dans lequel la liaison séparable (300) comprend un clipsage.

11. Système de surveillance corporelle (1) selon l'une quelconque des revendications précédentes comprenant en outre un guide (400) entre le premier module (100) et le deuxième module (200), le guide étant configuré pour permettre un retour naturel en position couplée.

12. Système de surveillance corporelle (1) selon la revendication 11, dans lequel le guide (400) comprend une forme tronconique (401) compris dans le premier module (100) et une ouverture complémentaire en entonnoir (402) dans la deuxième module (200), le guide permettant de définir une position intermédiaire, dans laquelle la forme tronconique (401) est engagée dans l'ouverture complémentaire en entonnoir (402).

13. Système de surveillance corporelle (1) selon la revendication 12 en combinaison avec la revendication 3, dans lequel le boitier (120) comprend une forme tronconique (401) et la capsule 220 comprend une ouverture traversante (224) évasée, de forme complémentaire.

14. Système de surveillance corporelle (1) selon l'une quelconque des revendications 1 à 13, dans lequel le seuil d'arrachement de la liaison séparable (300) a une valeur entre 30 et 400g, préférablement entre 70 et 250g.

15. Kit de surveillance corporelle (1), comprenant un système de surveillance corporelle (1) selon l'une quelconque des revendications 1 à 14 et un dock (400) configuré pour recevoir, au moins partiellement, le premier module (100) du dispositif de surveillance corporelle (1), le dock et le système de surveillance formant préférablement une liaison séparable aussi.
